# FASCICULE DE BREVET EUROPEEN

(11) **EP 3 176 093 B1**
(45) Date de publication et mention de la délivrance du brevet: **29.11.2017**
(21) Numéro de dépôt: 16199738.2
(22) Date de dépôt: 21.11.2016
(51) Int. Cl.: B64D 37/32, B65D 90/44, G01N 21/64

(54) **SYSTEME D'INERTAGE D'UN RESERVOIR DE CARBURANT D'UN AERONEF, ADAPTE POUR CALCULER LA QUANTITE D'OXYGENE PRESENTE DANS UN GAZ D'INERTAGE INJECTE DANS LEDIT RESERVOIR**
SYSTEM ZUR INERTISIERUNG EINES KRAFTSTOFFBEHÄLTERS EINES LUFTFAHRZEUGS, DAS IN DER LAGE IST, DIE IN DEM INERTISIERUNGSGAS, DAS IN DIESEN BEHÄLTER EINGESPRITZT WIRD, VORHANDENE SAUERSTOFFMENGE ZU BERECHNEN
SYSTEM FOR INERTING A FUEL TANK OF AN AIRCRAFT, SUITABLE FOR CALCULATING THE AMOUNT OF OXYGEN CONTAINED IN AN INERTING GAS INJECTED INTO SAID TANK

(30) Priorité: 03.12.2015 FR 1561818
(43) Date de publication de la demande: 07.06.2017
(73) Titulaire: Zodiac Aerotechnics, 42230 Roche La Moliere (FR)
(72) Inventeur: REYNARD, Bruno, 69340 Francheville (FR); DENAT, Frédéric, 42170 Saint-Just-Saint-Rambert (FR)
(74) Mandataire: Cabinet Laurent & Charras

(56) Documents cités:
- EP-A2- 2 712 807
- US-A- 5 047 627
- US-A1- 2005 286 054

## Description

### DOMAINE TECHNIQUE

La présente invention se rapporte au domaine technique des systèmes d'inertage pour réservoir de carburant d'un aéronef, tel qu'un avion, un hélicoptère ou analogue, et concerne plus particulièrement un système d'inertage adapté pour calculer la quantité d'oxygène présente dans un gaz d'inertage injecté dans un réservoir de carburant.

### ART ANTERIEUR

Dans le domaine de l'aéronautique, il est bien connu d'utiliser des systèmes d'inertage pour générer et introduire de l'azote, ou tout autre gaz neutre tel que le dioxyde de carbone par exemple, dans les réservoirs de carburant pour des raisons de sécurité afin de réduire le risque d'explosion desdits réservoirs.

Ces systèmes d'inertage sont également connus sous le terme OBIGGS selon l'acronyme anglo-saxon « On Board Inert Gas Generation Systems ».

Un système classique d'inertage de l'art antérieur comporte, d'une manière générale, un système embarqué de génération de gaz d'inertage dit OBIGGS alimenté en air, par exemple avec de l'air de purge détourné d'au moins un moteur. En effet, l'air de purge détourné d'au moins un moteur est actuellement le modèle le plus largement utilisé. Dans un tel système, la purge d'air est généralement déviée à partir d'un ou plusieurs moteurs à partir de l'ouverture dite de pression intermédiaire et/ou de l'ouverture dite de haute pression en fonction de la situation de vol. On notera que l'utilisation de purge d'air pour le conditionnement d'air est avantageuse car la purge d'air a une pression relativement élevée, ainsi qu'une température relativement élevée, de sorte que l'air peut être ajusté sur une large gamme de pressions et de températures désirées. Le système OBIGGS est couplé au réservoir de carburant de l'avion et sépare l'oxygène de l'air.

Le système OBIGGS est généralement constitué d'un module de séparation de l'air qui contient, par exemple, des membranes de zéolithe au travers desquelles un flux d'air est pressé. En raison des différents taux de transfert de masse de l'azote et de l'oxygène, le système divise le flux d'air de telle sorte qu'un flux d'air à forte teneur en azote et un flux d'air à forte teneur en oxygène sont obtenus. La fraction d'air enrichi en azote, considéré comme le gaz d'inertage, est acheminée dans les réservoirs de carburant de telle sorte que le mélange d'air et de vapeur de kérosène présent dans cet emplacement est déplacé et évacué hors des réservoirs. La fraction de l'air enrichi en oxygène peut être réintroduite dans la cabine des passagers après avoir été traitée avec des moyens appropriés et/ou dans la chambre de combustion des réacteurs afin d'améliorer la combustion. Les dispositifs nécessaires à cette opération tels que des compresseurs, des filtres, des modules de refroidissement à air, des eaux et analogues sont intégrés dans l'installation de gaz d'inertage.

Ainsi, lorsque le ratio entre le carburant et l'oxygène, dans la partie vide du réservoir est inférieur à la limite d'inflammation définie conformément aux exigences de la FAA selon l'acronyme anglo-saxon « Federal Aviation Administration » détaillées dans le document AC25.981-2A en date du 19 septembre 2008 et intitulé « FUEL TANK FLAMMABILITY REDUCTION MEANS » et ses annexes, aucune inflammation spontanée ne peut avoir lieu. De ce qui précède, rendre inerte un réservoir de carburant consiste notamment à maintenir le taux d'oxygène présent dans ledit réservoir sous un certain seuil, notamment 12%.

De ce qui précède, il est donc important de connaitre précisément la quantité d'oxygène présente dans le gaz rejeté par ledit module de séparation d'air, destiné à être injecté dans le ou les réservoirs de l'aéronef.

A cet effet, un système d'inertage connu intègre, d'une manière générale, un dispositif de mesure de la quantité d'oxygène agencé dans une conduite acheminant le gaz d'inertage dans le réservoir de carburant, cette conduite étant située en aval des moyens de séparation.

Dans l'état de la technique, il est connu un système d'inertage comprenant un dispositif de mesure comportant un boîtier définissant une chambre au travers de laquelle le gaz d'inertage peut circuler. Ce boîtier renferme un moyen de mesure pourvu d'une sonde au zirconium pour effectuer des mesures nécessaires dans ledit gaz pour connaitre la concentration en oxygène. La sonde au zirconium est notamment alimentée par une tension fixe et fonctionne avec une température relativement chaude.

L'inconvénient de ce type de système d'inertage est qu'il intègre des moyens de mesure de la quantité d'oxygène fonctionnant à chaud et à base d'un matériau métallique. Le système d'inertage est donc encombrant et alourdi, et met en oeuvre une connectique relativement complexe.

De plus, ce type de dispositif de mesure est sensible aux conditions environnementales, et la mesure donnée par celui-ci peut dériver de manière incontrôlée. En effet, les mesures effectuées par la sonde au zirconium sont variables en fonction des conditions environnementales d'utilisation de ladite sonde, et notamment en fonction de la température et de la pression dans laquelle est maintenu le moyen de mesure.

Enfin, la mesure effectuée par la sonde dérive d'une manière aléatoire dans le temps du fait du vieillissement de l'élément sensible à base de zirconium. Le document EP2712807 décrit un système d'inertage d'un réservoir de carburant d'un aéronef, comprenant un générateur de gaz d'inertage alimenté avec de l'air de purge détourné d'au moins un moteur et/ou de l'air d'une cabine de passagers; des moyens de distribution du gaz d'inertage dans le réservoir de carburant, reliés au générateur et intégrant un dispositif de mesure de la quantité d'oxygène présente dans ledit gaz d'inertage et un capteur de débit agencé dans les moyens de distribution et en contact avec le gaz d'inertage.

### EXPOSE DE L'INVENTION

L'un des buts de l'invention est donc de remédier à ces inconvénients en proposant un système d'inertage d'un réservoir de carburant d'un aéronef qui permet, en outre, de calculer la quantité d'oxygène présente dans un gaz d'inertage injecté dans le réservoir de carburant, d'une manière simple, fiable et précise dans le temps, sans impacter de manière significative le poids dudit système.

Un autre objectif de l'invention est notamment de fournir un tel système d'inertage dont la mesure de la quantité d'oxygène est moins sensible aux conditions environnementales, de sorte à limiter la dérive de sa mesure, voire même à la supprimer.

A cet effet, il a été mis au point un système d'inertage d'au moins un réservoir de carburant d'un aéronef, tel qu'un avion ou un hélicoptère par exemple, ou analogue comprenant :
- au moins un générateur de gaz d'inertage alimenté avec de l'air de purge détourné d'au moins un moteur et/ou de l'air d'une cabine de passagers ;
- des moyens de distribution du gaz d'inertage dans le ou les réservoirs de carburant, reliés au générateur de gaz d'inertage et intégrant un dispositif de mesure de la quantité d'oxygène présente dans ledit gaz d'inertage.

Conformément à l'invention, le dispositif de mesure comprend :
- un capteur comprenant matériau phosphorescent, agencé dans les moyens de distribution et en contact avec le gaz d'inertage ;
- une source lumineuse illuminant le matériau phosphorescent ;
- des moyens de mesure de la phosphorescence du matériau phosphorescent ;
- des moyens de calcul de la quantité d'oxygène présente dans le gaz d'inertage en fonction de l'atténuation de la phosphorescence mesurée qui est directement liée à la quantité d'oxygène dans le gaz d'inertage.

En effet, certains matériaux ont la propriété d'émettre des photons lors de leur passage d'un état excité à un niveau d'énergie inférieur. L'état excité est obtenu, par exemple, par absorption d'une radiation électromagnétique émise par la source lumineuse. Il s'ensuit un effet d'atténuation ou d'extinction de la phosphorescence, lié à la présence d'une molécule dans le milieu réactionnel, par exemple à la présence d'oxygène. Plus la phosphorescence du matériau est atténuée rapidement, plus la quantité d'oxygène présente dans le gaz d'inertage environnant le matériau phosphorescent est élevée. En fonction de la constante de temps de l'atténuation de la phosphorescence d'un matériau, les moyens de calcul sont aptes à calculer la quantité d'oxygène présente dans le gaz. En pratique, les moyens de calcul déterminent la variation de l'intensité de la phosphorescence en fonction du temps et calculent une constante de décroissance. C'est cette constante de décroissance qui permet de calculer la quantité d'oxygène présente dans le gaz en fonction du matériau phosphorescent utilisé.

Ainsi, le système d'inertage selon l'invention est plus simple à mettre en oeuvre car il ne nécessite pas nécessairement de chambre spécifique à la mesure, et ne créé pas de point chaud dans le système. Le dispositif de mesure du système d'inertage fonctionne à froid et en temps réel. De plus, il est moins encombrant, plus léger et moins complexe que les systèmes de l'art antérieur précédemment décrits.

Connaitre la quantité d'oxygène présente dans le gaz d'inertage en sortie du générateur de gaz d'inertage permet notamment d'effectuer un diagnostic dudit générateur afin de vérifier son état de fonctionnement, ses performances et prévoir ou non un remplacement ou une maintenance sur ledit générateur.

Selon une forme de réalisation particulière du système d'inertage, le capteur est relié par l'intermédiaire de fibres optiques, d'une part, à la source lumineuse et, d'autre part, aux moyens de mesure, ladite source lumineuse et lesdits moyens de mesure étant agencés hors des moyens de distribution du gaz d'inertage.

De cette manière, la source lumineuse est déportée du matériau phosphorescent, et illumine le matériau phosphorescent au moyen des fibres optiques. Il en est de même pour les moyens de mesure qui peuvent être aussi déportés car ils observent la phosphorescence du matériau par l'intermédiaire des fibres optiques. Les fibres optiques sont souples, légères et peuvent être courbées ce qui facilite considérablement le montage du système. Le système n'est ni encombré ni alourdi.

Selon une forme de réalisation particulière, le matériau phosphorescent comprend une matrice polymère et un composé phosphorescent et se présente par exemple sous la forme d'une pastille.

Avantageusement, la source lumineuse comprend au moins une diode électroluminescente.

Dans le cas où le matériau phosphorescent utilisé présente également une fluorescence en réponse à l'illumination par la source lumineuse, et afin que la mesure soit la plus précise possible, le capteur comprend des moyens pour éliminer des émissions fluorescentes ainsi que la réflexion de la lumière incidente provenant de la source lumineuse sur ledit matériau phosphorescent.

Les moyens de mesure sont de tout type approprié, et comprennent par exemple un photo-détecteur.

### DESCRIPTION SOMMAIRE DES FIGURES

D'autres avantages et caractéristiques ressortiront mieux de la description qui va suivre, donnée à titre d'exemple non limitatif, du dispositif de mesure selon l'invention, à partir des dessins annexés dans lesquels :
- la figure 1 illustre un schéma de principe d'un système d'inertage selon l'invention ;
- la figure 2 est une représentation schématique illustrant l'agencement du dispositif de mesure par rapport aux moyens de distribution du gaz d'inertage ;
- la figure 3 est une représentation schématique d'une forme de réalisation du dispositif de mesure du système d'inertage selon l'invention ;
- la figure 4 illustre un graphique représentant, pour un matériau phosphorescent déterminé, la constante de temps de l'atténuation de la phosphorescence dudit matériau phosphorescent lorsqu'il est illuminé par la source lumineuse, en fonction de la quantité d'oxygène présente dans le gaz d'inertage.

### DESCRIPTION DETAILLEE DE L'INVENTION

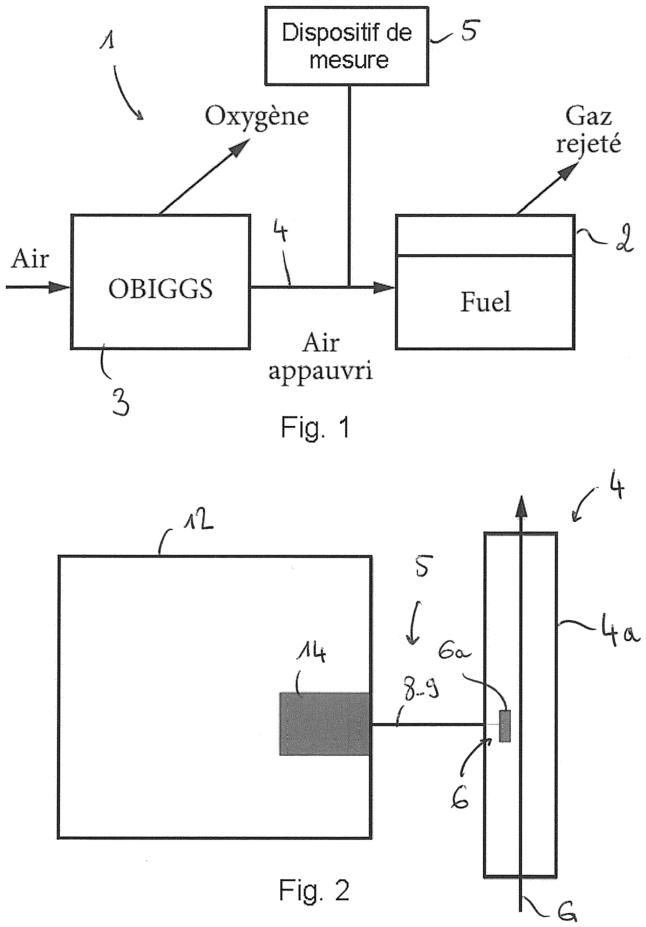

En référence à la figure 1, l'invention concerne un système d'inertage (1) d'un ou plusieurs réservoirs (2) d'un aéronef pour générer et introduire de l'azote, ou tout autre gaz neutre tel que le dioxyde de carbone par exemple, dans le ou lesdits réservoirs (2) de carburant pour des raisons de sécurité afin de réduire le risque d'explosion desdits réservoirs (2).

Le système d'inertage (1) comprend, d'une manière générale, un générateur (3) embarqué de gaz d'inertage dit OBIGGS, selon l'acronyme anglo-saxon « *On Board Inert Gas Generation Systems* », alimenté en air, par exemple avec de l'air de purge détourné d'au moins un moteur et/ou de l'air provenant d'une cabine de passagers de l'aéronef. Le générateur (3) de gaz d'inertage se présente par exemple sous la forme de membranes de zéolithe au travers desquelles de l'air est pressé de manière à obtenir, d'une part, un gaz d'inertage à forte teneur en azote et, d'autre part, un gaz à forte teneur en oxygène.

Le système d'inertage (1) comprend en outre des moyens de distribution (4), telles que des conduites par exemple, du gaz d'inertage dans le ou les réservoirs (2) de carburant, reliés au générateur (3) de gaz d'inertage. Le gaz d'inertage injecté vise à rendre inerte le ou les réservoirs (2) de carburant, c'est-à-dire qu'ils permettent de réduire le taux d'oxygène présent dans le ou lesdits réservoirs (2), et notamment à maintenir ce taux sous un certain seuil, de préférence inférieure à 12%.

Selon l'invention, le système d'inertage (1) comprend un dispositif de mesure (5) de la quantité d'oxygène présente dans le gaz d'inertage injecté dans le ou les réservoirs (2).

Plus précisément, et en référence à la figure 2, le dispositif de mesure (5) comprend un capteur (6) comprenant un matériau phosphorescent (7). Le capteur (6) est agencé à l'intérieur des moyens de distribution (4), tel que dans une conduite de gaz (4a) ou dans une vanne, en aval du générateur (3) de gaz d'inertage et en amont des réservoirs (2), et de telle sorte que le matériau phosphorescent (7) soit en contact avec le gaz d'inertage. La flèche (G) qui traversent la conduite (4a) représente le passage du gaz d'inertage

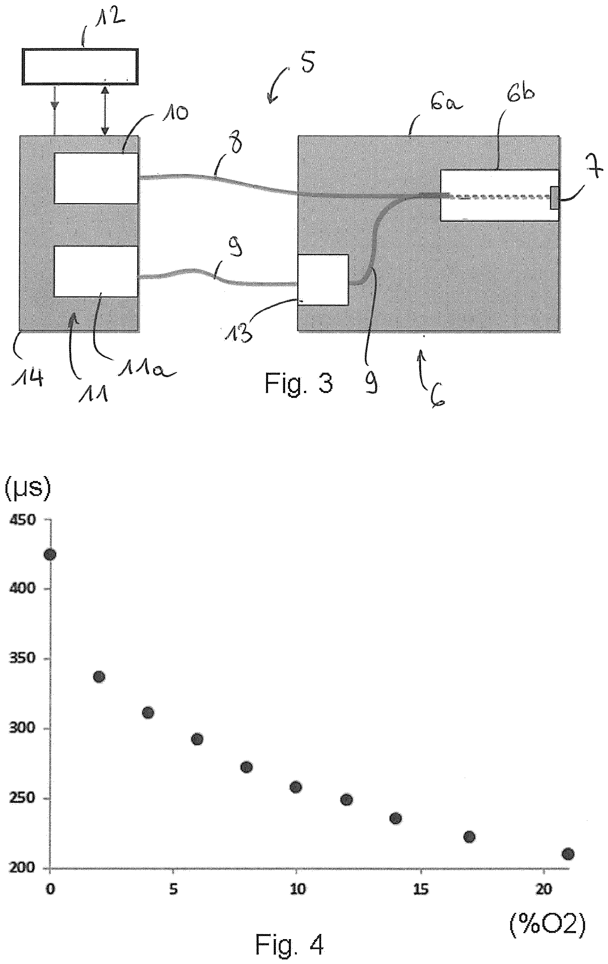

En référence à la figure 3, et selon une forme de réalisation particulière, le capteur (6) se présente sous la forme d'une sonde (6a), immergée dans les moyens de distribution (4) et en contact avec le gaz d'inertage. Cette sonde (6a) intègre un adaptateur (6b) sur lequel est fixé, à une extrémité, le matériau phosphorescent (7). L'adaptateur (6b) est lui-même positionné à une extrémité de la sonde (6a) pour mettre en contact le matériau phosphorescent (7) avec le gaz d'inertage.

Une autre extrémité de l'adaptateur (6b) comprend des première et deuxième fibres optiques (8, 9), agencées en alignement avec ledit matériau phosphorescent (7), c'est-à-dire dont les extrémités sont positionnées au droit dudit matériau phosphorescent (7). La ou les premières fibres optiques (8) sont reliées à une source lumineuse (10) comportant au moins une diode électroluminescente pour illuminer ledit matériau phosphorescent (7). La ou les deuxièmes fibres optiques (9) sont reliées à des moyens de mesure (11) de la phosphorescence du matériau phosphorescent (7) qui se présentent par exemple sous la forme d'un photo-détecteur (11a).

De cette manière, la source lumineuse (10) illumine pendant un laps de temps de 0.1 ms à quelques ms, par exemple 3ms, de sorte à faire passer le matériau phosphorescent (7) dans un état excité. Après l'illumination du matériau phosphorescent (7) et lors du passage du matériau phosphorescent (7) à un état d'énergie inférieur, celui-ci émet des photons ce qui révèle sa phosphorescence. La phosphorescence est mesurée par le photo-détecteur (11a) au moyen des deuxièmes fibres optiques (9). L'atténuation de la phosphorescence mesurée par le photo-détecteur (11a), qui est directement liée à la quantité d'oxygène dans le gaz d'inertage, est calculée par des moyens de calcul (12).

Les moyens de calcul (12) intègrent des moyens de traitement aptes à éliminer, d'une part, des émissions fluorescentes dans le cas où le matériau phosphorescent (7) utilisé est aussi fluorescent, et, d'autre part, la réflexion de la lumière incidente de la source lumineuse (10) sur ledit matériau phosphorescent (7). Selon une autre forme de réalisation particulière, le photo-détecteur (11a) observe la phosphorescence du matériau phosphorescent (7) au travers d'un filtre optique (13) apte à réaliser ces fonctions d'élimination.

De préférence, le photo-détecteur (11a) et la source lumineuse (10) font partie d'un module (14) connecté aux moyens de calcul (12), tel qu'un calculateur, permettant, d'une part, d'alimenter électriquement le module (14) et, d'autre part, de calculer la quantité d'oxygène dans le gaz d'inertage en fonction des données d'intensité de la phosphorescence reçues du photo-détecteur (11a). Les moyens de calcul (12) déterminent la variation de l'intensité de la phosphorescence en fonction du temps et calculent une constante de décroissance. C'est cette constante de décroissance qui permet de calculer la quantité d'oxygène présente dans le gaz en fonction du matériau phosphorescent (7) utilisé.

S'agissant du matériau phosphorescent (7), plusieurs compositions sont possibles. L'essentiel réside dans le fait que ledit matériau passe d'un état excité phosphorescent lorsqu'il est illuminé par la source lumineuse (10), à un état normal désexcité non phosphorescent.

En pratique, le photo-détecteur (11a) mesure ladite phosphorescence et les moyens de calcul (12) permettent de calculer l'atténuation de la phosphorescence, à savoir la constante de temps de l'atténuation de la phosphorescence qui dépend notamment de la quantité d'oxygène présente dans le gaz d'inertage.

De cette manière, il est possible de générer, au préalable, un graphique, tel que représenté sur la figure 4, identifiant la constante de temps de l'atténuation de la phosphorescence d'un matériau phosphorescent (7) déterminé en présence d'une quantité d'oxygène connue. Le matériau phosphorescent (7) déterminé est caractérisé par une certaine composition chimique et une certaine épaisseur.

De cette manière, au moyen de ce graphique, il est possible de déterminer la quantité d'oxygène présente dans le gaz d'inertage en fonction de la constante de temps de l'atténuation de la phosphorescence calculée par les moyens de calcul (12).

Pour l'application considérée, consistant à maintenir le taux d'oxygène présent dans le gaz d'inertage injecté dans un réservoir (2) de carburant sous un certain seuil, notamment inférieur à 12%, le matériau phosphorescent (7) doit présenter une atténuation significative de sa phosphorescence en présence d'une telle quantité d'oxygène. Ainsi, il est préférable d'avoir un matériau phosphorescent (7) ayant une forte variation de sa vitesse d'atténuation de sa phosphorescence en présence de 5% à 15% d'oxygène dans le gaz, et de préférence en présence de 8% à 12% d'oxygène. L'atténuation doit être précise et rapide, notamment dans un délai compris entre 1 et 5 min, compte tenu de l'application liée à la sécurité des aéronefs.

Par ailleurs, la quantité d'oxygène dans le gaz qui est fonction de l'atténuation de la phosphorescence d'un matériau phosphorescent (7), dépend également de la température et de la pression partielle d'oxygène présente dans le gaz. Ainsi, les moyens de calcul (12) sont aussi couplés à des moyens permettant de mesurer la température et/ou la pression partielle d'oxygène pour permettre le calcul de la quantité d'oxygène.

Dans un exemple de réalisation préféré, compatible avec l'application concernée en ce qui concerne la vitesse d'atténuation de la phosphorescence, à savoir le temps de réponse du matériau et la plage de la quantité d'oxygène à laquelle l'atténuation est la plus significative, le matériau phosphorescent (7) utilisé se présente par exemple sous la forme d'une pastille de quelques mm à environ 1 cm de diamètre et de 150 µm d'épaisseur. La pastille comprend une matrice polymère comprenant un composé phosphorescent. Le composé phosphorescent peut être un complexe de porphyrine de métal du groupe 10, tel qu'un complexe de porphyrine de palladium ou de platine.

A titre d'exemple, le composé phosphorescent peut être :
- du 5,10,15,20-Tétrakis(2,3,4,5,6-pentafluorophényl)porphyrine-Pd(II) : 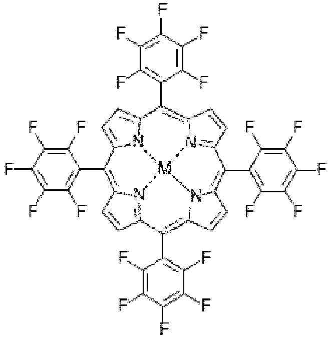 Avec M = Pd
- du 2,3,7,8,12,13,17,18-Octaéthylporphyrine-Pd(II) ou du 2,3,7,8,12,13,17,18-Octaéthylporphyrine-Pt(II) : 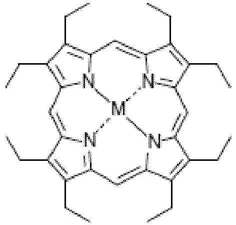 Avec M = Pd ou Pt
- du Meso-Tétraphénylporphyrine-Pd(II) ou du Meso-Tétraphénylporphyrine-Pt(II) : 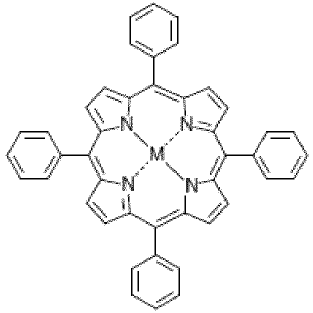 Avec M = Pd ou Pt
- du 5,10,15,20-Tétrakis(4-hydroxyméthylphényl)porphyrine-Pt(II) : 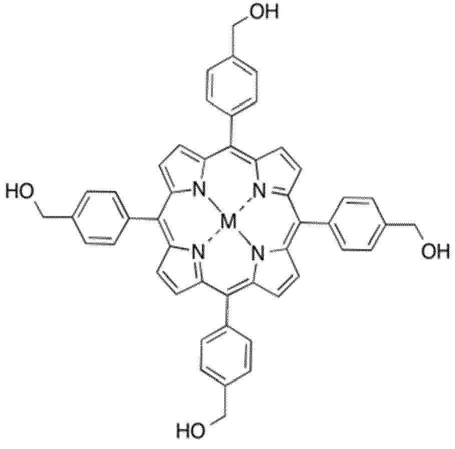 Avec M = Pt

D'autres composés peuvent être utilisés sans sortir du cadre de l'invention, tels que notamment des composés organiques du type :

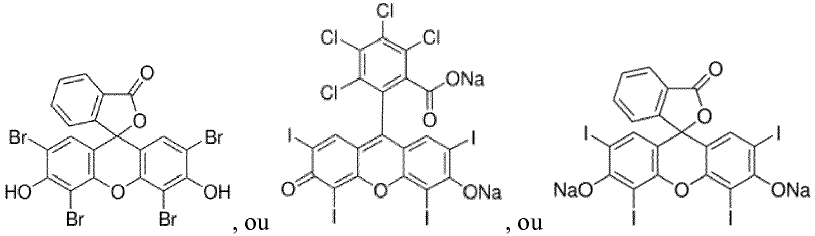

La matrice polymère est mélangée avec le composé phosphorescent. Il convient de choisir la matrice polymère en fonction de l'application considérée. En d'autres termes, la matrice polymère doit présenter une certaine résistance aux vapeurs de kérosène et être liée au composé phosphorescent. Idéalement, on peut avoir une ou plusieurs liaisons covalentes entre les molécules de la matrice et du composé phosphorescent.

La matrice polymère peut être du polyuréthane obtenu par la réaction d'un composé comprenant au moins deux fonctions isocyanate et un composé comprenant au moins deux fonctions alcool.

A titre d'exemple la matrice polymère peut être un polyuréthane obtenu par la réaction entre :
- un PDTI (poly(propylene glycol),tolylene 2, 4-diisocyanate terminated) : 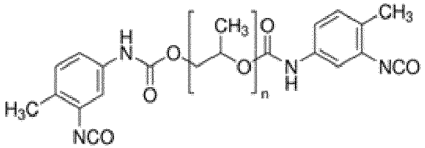 Et,
- un TMP (triméthylolpropane) :

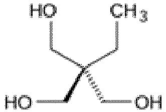

Un surfactant peut aussi être ajouté au mélange.

Selon un autre exemple de réalisation, la matrice polymère peut être un polyuréthane obtenu par la réaction entre :
- TMP (triméthylolpropane) :
   Et,
- un PHD (Poly(hexaméthylène diisocyanate) :

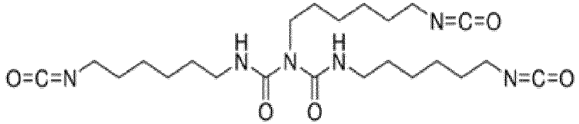

Un composé phosphorescent est ensuite intégré au polyuréthane pour former le matériau phosphorescent (7) selon la présente invention.

De ce qui précède, l'invention fournit un système d'inertage (1) d'un réservoir de carburant d'un aéronef, capable de calculer la quantité d'oxygène présente dans le gaz d'inertage injecté dans le ou les réservoirs (2). Le calcul de la quantité d'oxygène est réalisé d'une manière simple, fiable et précise dans le temps, sans impacter de manière significative le poids dudit système car il met en oeuvre des fibres optiques légères. Le système met en oeuvre une mesure par luminescence qui n'implique pas de montée en température du dispositif de mesure (5). La mesure se fait à froid, sans risque pour le système d'inertage (1) et l'aéronef. La phosphorescence du matériau n'est pas altérable, ni sensible aux conditions environnementales, de sorte que la mesure ne dérive pas au cours du temps.

Le calcul de la quantité d'oxygène permet ensuite d'agir sur le générateur (3) de gaz d'inertage en fonction de la valeur calculée pour générer un gaz d'inertage plus ou moins chargé en oxygène pour l'injection dans le ou les réservoirs (2) de carburant.

L'invention permet enfin de connaitre la qualité du gaz d'inertage en sortie du générateur de gaz d'inertage et permet ainsi d'effectuer un diagnostic dudit générateur afin de vérifier son état de fonctionnement, ses performances et prévoir ou non un remplacement ou une maintenance sur ledit générateur.

## Revendications

1. Système d'inertage (1) d'au moins un réservoir de carburant (2) d'un aéronef, ledit système comprenant :
- au moins un générateur (3) de gaz d'inertage alimenté avec de l'air de purge détourné d'au moins un moteur et/ou de l'air d'une cabine de passagers ;
- des moyens de distribution (4) du gaz d'inertage dans le ou les réservoirs (2) de carburant, reliés au générateur (3) de gaz d'inertage et intégrant un dispositif de mesure (5) de la quantité d'oxygène présente dans ledit gaz d'inertage,
- un capteur (6) comprenant un matériau phosphorescent (7), agencé dans les moyens de distribution (4) et en contact avec le gaz d'inertage ;
- une source lumineuse (10) illuminant le matériau phosphorescent (7) ;
- des moyens de mesure (11) de la phosphorescence du matériau phosphorescent (7) ;
- des moyens de calcul (12) de la quantité d'oxygène présente dans le gaz d'inertage en fonction de l'atténuation de la phosphorescence mesurée qui est directement liée à la quantité d'oxygène dans le gaz d'inertage.

2. Système d'inertage (1) selon la revendication 1, ***caractérisé* en ce que** le capteur (6) est relié par l'intermédiaire de fibres optiques, d'une part, à la source lumineuse (10) et, d'autre part, aux moyens de mesure (11), ladite source lumineuse (10) et lesdits moyens de mesure (11) étant agencés hors des moyens de distribution (4) du gaz d'inertage.

3. Système d'inertage (1) selon l'une quelconque des revendications 1 à 2, ***caractérisé* en ce que** le matériau phosphorescent (7) comprend une matrice polymère et un composé phosphorescent.

4. Système d'inertage (1) selon l'une des revendications 1 à 3, ***caractérisé* en ce que** la source lumineuse (10) comprend au moins une diode électroluminescente.

5. Système d'inertage (1) selon l'une quelconque des revendications 1 à 4, ***caractérisé* en ce que** le dispositif de mesure (5) comprend des moyens pour éliminer des émissions fluorescentes et la réflexion de la lumière incidente provenant de la source lumineuse (10) sur ledit matériau phosphorescent (7).

6. Système d'inertage (1) selon l'une quelconque des revendications 1 à 5, ***caractérisé* en ce que** les moyens de mesure (11) comprennent un photo-détecteur (11a).

## Patentansprüche

1. System zur Inertisierung (1) mindestens eines Treibstofftanks (2) eines Luftfahrzeugs, wobei das System Folgendes umfasst:
- mindestens ein Erzeugungsgerät (3) für Inertschutzgas, wobei dieses mit Spülluft gespeist wird, die aus mindestens einem Triebwerk und/oder der Luft einer Passagierkabine abgezapft wird;
- Mittel (4) zur Abgabe des Inertschutzgases in den oder die Treibstofftanks (2), wobei diese mit dem Erzeugungsgerät (3) für Inertschutzgas verbunden sind und eine Vorrichtung (5) zum Messen der Sauerstoffmenge beinhalten, welche in dem Inertschutzgas vorliegt,
- einen Sensor (6), der ein phosphoreszierendes Material (7) umfasst, wobei er in den Abgabemitteln (4) angeordnet ist und mit dem Inertschutzgas in Kontakt ist;
- eine Lichtquelle (10), die das phosphoreszierende Material (7) beleuchtet;
- Mittel (11) zum Messen der Phosphoreszenz des phosphoreszierenden Materials (7);
- Mittel (12) zum Berechnen der Menge an Sauerstoff, die in dem Inertschutzgas vorliegt, in Abhängigkeit von der Abschwächung der gemessenen Phosphoreszenz, welche unmittelbar mit der Sauerstoffmenge in Verbindung steht, die in dem Inertschutzgas vorliegt.

2. Inertisierungssystem (1) nach Anspruch 1, **dadurch *gekennzeichnet*, dass** der Sensor (6) über Lichtwellenleiter einerseits mit der Lichtquelle (10) und andererseits mit den Messmitteln (11) verbunden ist, wobei die Lichtquelle (10) und die Messmittel (11) außerhalb der Mittel (4) zur Abgabe des Inertschutzgases angeordnet sind.

3. Inertisierungssystem (1) nach einem beliebigen der Ansprüche 1 bis 2, **dadurch *gekennzeichnet*, dass** das phosphoreszierende Material (7) eine Polymermatrix und eine phosphoreszierende Verbindung umfasst.

4. Inertisierungssystem (1) nach einem der Ansprüche 1 bis 3, **dadurch *gekennzeichnet*, dass** die Lichtquelle (10) mindestens eine Leuchtdiode umfasst.

5. Inertisierungssystem (1) nach einem beliebigen der Ansprüche 1 bis 4, **dadurch *gekennzeichnet*, dass** die Messvorrichtung (5) Mittel zum Ausblenden der ausgesandten Fluoreszenz sowie der Reflexion des Lichts umfasst, welches aus der Lichtquelle (10) stammt und auf das phosphoreszierende Material (7) trifft.

6. Inertisierungssystem (1) nach einem beliebigen der Ansprüche 1 bis 5, **dadurch *gekennzeichnet*, dass** die Messmittel (11) einen Photodetektor (11a) umfassen.

## Claims

1. An inerting system (1) for inerting at least one fuel tank (2) of an aircraft, said system comprising:
- at least one inerting gas generator (3) fed with bleed air diverted from at least one engine and/or with air from a passenger cabin;
- distribution means (4) for distributing inerting gas to the fuel tank(s) (2), connected to the inerting gas generator (3) and incorporating a measurement device (5) for measuring the quantity of oxygen present in said inerting gas.
- a sensor (6) including a phosphorescent material (7), arranged in the distribution means (4) and in contact with the inerting gas;
- a light source (10) illuminating the phosphorescent material (7);
- measurement means (11) for measuring the phosphorescence of the phosphorescent material (7); and
- computation means (12) for computing the quantity of oxygen present in the inerting gas as a function of the attenuation of the phosphorescence as measured that is directly related to the quantity of oxygen in the inerting gas.

2. An inerting system (1) according to claim 1, **characterized in that** the sensor (6) is connected via optical fibers firstly to the light source (10) and secondly to the measurement means (11), said light source (10) and said measurement means (11) being arranged outside the distribution means (4) for distributing the inerting gas.

3. An inerting system (1) according to claim 1 or claim 2, **characterized in that** the phosphorescent material (7) comprises a polymer matrix and a phosphorescent compound.

4. An inerting system (1) according to any one of claims 1 to 3, **characterized in that** the light source (10) comprises at least one light-emitting diode.

5. An inerting system (1) according to any one of claims 1 to 4, **characterized in that** the measurement device (5) includes means for removing fluorescent emissions and the reflection of the incident light coming from the light source (10) off said phosphorescent material (7).

6. An inerting system (1) according to any one of claims 1 to 5, **characterized in that** the measurement means (11) include a photo-detector (11a).
